(19) European Patent Office

Europäisches Patentamt

European Patent Office

Office européen des brevets

(11) **EP 1 047 018 A1**

(12) **EUROPEAN PATENT APPLICATION**

(43) Date of publication:
**25.10.2000 Bulletin 2000/43**

(51) Int. Cl.$^7$: **G06T 7/00**, A61B 6/00

(21) Application number: **00303309.9**

(22) Date of filing: **19.04.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **22.04.1999 FR 9905128**

(71) Applicant: **GE MEDICAL SYSTEMS SA**
**78533 Buc Cedex (FR)**

(72) Inventors:
• **Muller, Serge**
**78280 Guyancourt (FR)**

• **Rick, Andreas**
**78370 Plaisir (FR)**
• **Bothorel, Sylvie**
**92100 Boulogne-Billancourt (FR)**

(74) Representative:
**Pedder, James Cuthbert**
**GE London Patent Operation,**
**Essex House,**
**12/13 Essex Street**
**London WC2R 3AA (GB)**

(54) **Method of localization and three-dimensional representation of elements of interest of an organ**

(57)    A method is described of localization and representation of elements of interest of an organ from a plurality of digital images taken at different angles, stereotactic images. The elements of interest present in those digital stereotactic images are determined automatically, the three-dimensional coordinates of each element of interest are determined and the elements of interest are displayed in three dimensions.

EP 1 047 018 A1

**Description**

The invention concerns the localization and three-dimensional representation of elements of interest of an organ from a set of stereotactic images of the organ.

Although the invention applies to any set of stereotactic images of any organ, it is particularly useful in the medical field and, notably, m mammography upon a cytological analysis or histological analysis in which it is desired to test a part of the breast.

Following a screening examination, when radiological signs are discovered in a patient which might be associated with a cancer, microcalcifications in particular, an additional diagnostic examination is made, which can in turn be supplemented by an interventional radiology examination. In the course of the examination, the physician may want to test, by means of a needle, an area of the breast having cells of a suspicious character, with a view to a cytological analysis. This procedure is characterized as cytopuncture. The physician may also wish to test with a needle organic tissues in an area of the breast of a suspicious character, with a view to an histological analysis. . This procedure is characterized as microbiopsy. In general, one speaks of needle biopsy.

This puncture or needle biopsy procedure, that is, testing cells or organic tissues containing elements of interest necessitates a precise knowledge of those elements in three dimensions. Hereafter, "element of interest" describes a microcalcification or any other internal element of an organ likely to interest an operator.

A stereotactic examination makes it possible to access a given point of an organ with great precision, in the order of a millimeter, from at least two two-dimensional projections of that organ acquired at two angles of incidence, for example, opposite on both sides from normal on the plane of the image receiver delivering the projections.

With knowledge, on the one hand, of the position of projection of that point in the plane of each stereotactic image obtained and, on the other, of the geometry of the stereotactic camera having resulted in the construction of the two images, it is possible to compute the exact spatial position of that point in the three-dimensional organ by trigonometric calculation.

In summary, a stereotactic examination requires:

taking at least two images of the object at different angles,

knowing perfectly the geometry of acquisition of the stereotactic system or being able to reconstruct it from markers placed in the field of view and visible on the projection images, and

being able to localize on the different stereotactic images obtained the site of projection of the element of interest chosen.

The first two points pose no major problem.

At present in mammography, the stereotactic examination is carried out by means of a mammography machine equipped with a stereotactic camera. The mammography machine comprises an X-ray tube, situated at the end of a first arm which moves on an axis and emits x-radiation to a receiver situated at the end of another arm. Placed between the tube and the receiver are a breast support plate, or patient's support, on one side, and a compression plate, on the other, holding the breast in place for mammograms. The image receiver can be a digital receiver, such as a CCD camera, for example, delivering digitized stereotactic images. The stereotactic pictures necessitate rotation of the X-ray tubes around the breast support and compression plates in two successive orientations, for example, opposite on both sides of its initial position perpendicular to the plane of the image receiver.

With the digitized stereotactic images obtained, the localization of an element of interest on the different images employs a matching of homologous regions of interest in two different stereotactic images to a same element of interest situated in the breast.

The stereotactic examination therefore makes it possible to localize elements of interest by determining their three-dimensional coordinates. Those coordinates are then transmitted to a positioner, otherwise known as needle holder, making it possible to guide the puncture needle in the breast.

Heretofore, a cytopuncture operation, for example, required:

- a realization of stereotactic images;

- a selection of a region of interest a microcalcification, for example, on the two-dimensional stereotactic images either manually by the radiologist or automatically by an automatic selection process;

- an algorithm of localization in order to determine the coordinates of microcalcification; and

- the performance of puncture or needle biopsy according to the coordinates so determined.

This method is disclosed in French Patent No. 2,751,109. Briefly, this method involves the localization of an element of interest contained in a three-dimensional object from positions of homologous regions of interest corresponding to the element of interest and appearing in a set of stereotactic images of the object. A selection is first made in a first stereotactic image of a first region of interest called "target". Then, the first region is matched with a second region of interest homologous with the first and appearing in a second stereotactic image. The stereotactic images being digitized, a target pixel is selected in the target region of interest. In the matching stage a target window of chosen dimensional characteristics and containing the target region of interest is generated around the target pixel selected. A set of pixels is then determined in the second image according to a predetermined selection criterion and a second window of the same dimensional characteristics as the target window is generated around each pixel selected. A correlation treatment is carried out between the gray levels of the pixels of each second window and the gray levels of the pixels of the target window, so as to obtain a correlation value for each second window. The region of interest homologous with the target region of interest is then identified by analysis of the set of correlation values thus obtained, so as to minimize the risks of error of matching between the homologous regions of interest.

This known procedure involves the selection of the region of interest on two-dimensional images. If this selection is made manually, the operator chooses an element of interest which, in his/her opinion, represents a microcalcification. In fact a stereotactic image contains several elements of the image among which some represent microcalcifications and are due to noise. It is often difficult to distinguish the microcalcifications from the noise, for the stereotactic images do not always possess a quality sufficient to be able to distinguish, elements of interest. Even an automatic process of selection on stereotactic images does not always make it possible to select a region of interest correctly, especially when several elements form a cluster.

By selecting a target point from those two projections in two images, the physician does not know what position that target point occupies in the volume to be tested, in relation to other elements of interest. It would be of particular interest to puncture or perform a needle biopsy in an element of interest situated on the periphery or in the center of a group or cluster of elements of interest such as microcalcifications, for example.

The invention is aimed at introducing a solution to that problem by bringing in the selection stage as late as possible in the process.

The invention reduces appreciably the risk of error in selection of the region to be punctured by obtaining a three-dimensional representation of the group of elements of interest.

The invention therefore proposes a method of localization and representation of elements of interest of an organ from a plurality of digital images taken at different angles, in which the elements of interest present in the digital images are determined, the three-dimensional coordinates of each element of interest are then determined, the elements of interest are next displayed in three dimensions and, finally, one or more elements of interest are selected manually.

The invention will now be described in greater detail, by way of example, with reference to the drawings, in which:-

- Figure 1 is a flow chart of a process according to the invention of obtaining stereotactic images up to transfer of the three-dimensional coordinates: to the positioner; and

- Figure 2 is a schematic view of the process.

This method of the invention involves, in fad, four different stages, in which the first stage is that of determination of the elements of interest present in the two-dimensional stereotactic images. While heretofore a target region of interest was selected on a first image, the present invention provides for detection of the group of elements of interest present in each image. The physician is no longer required to choose, on one or more projection images of the breast, a target element of interest. The group of elements of interest, such as the projection of microcalcifications, is determined in each image.

The second stage involves a method of localization of the microcalcifications so determined. This stage makes it possible, in fact to obtain the three-dimensional coordinates of each microcalcification.

The third stage corresponds to the display of those microcalcifications in a three-dimensional space.

The last stage is that of selection, by choice of the operator, of a particular element of interest in the three-dimensional space.

According to a method of use of the invention, the elements of interest are advantageously determined by means of an automatic process of detection of elements of interest. An algorithm making it possible to distinguish the microcalcifications from noise is applied.

The automatic detection process advantageously employs a mathematical model of the acquisition chain and of the organ to be X-rayed and a noise model. This process preferably takes into account the parameters of acquisition of the acquisition chain and can use a top-hat transformation.

In general, the three-dimensional coordinates of each element of interest detected are determined by making an automatic matching from at least two of the digital images. For this purpose, stereotactic images are used, in which the same microcalcification has been determined thanks, notably, to matching.

The three-dimensional coordinates of one or more selected elements of interest are advantageously sent to a needle positioner. The three-dimensional coordinates of each selected element of interest correspond to the coordinates of a target point of the breast on which a puncture or needle biopsy can be made.

Furthermore, following determination of the elements of interest, a reconstruction of three-dimensional images of an element of interest can be made from two-dimensional images in which the element of interest has been detected, and its three-dimensional form can then be displayed. In fact when the microcalcifications are determined, one or more clusters of them, in particular, might be involved. In this case, one might just want to study its form without necessarily knowing its coordinates. Starting with stereotactic images in which a same cluster of microcalcifications is displayed, a three-dimensional reconstruction algorithm is applied, making it possible to observe on a screen the shape of the cluster, in order to thus avoid puncture in a part not containing any element of interest, for example.

According to an embodiment of the invention, one can use a system of display on a screen integrating a rotation function, in order to distinguish the alignment of the elements of interest upon a diagnosis, and a cursor function, in order to select the element to be punctured on the screen. Image processing software is therefore advantageously used, in which the three-dimensional coordinates of the microcalcifications previously determined are sent. The software makes it possible to represent the microcalcifications in space and possible alignments can thus be observed. These alignments can correspond to milk ducts on a mammogram. The three-dimensional representation contains more information than a two-dimensional image and therefore offers an enriched diagnostic support. By means of software functions, it is then possible to observe the microcalcifications at different angles and to select at that time the microcalcification to be punctured. The coordinates can be directly sent by the cursor to the positioner.

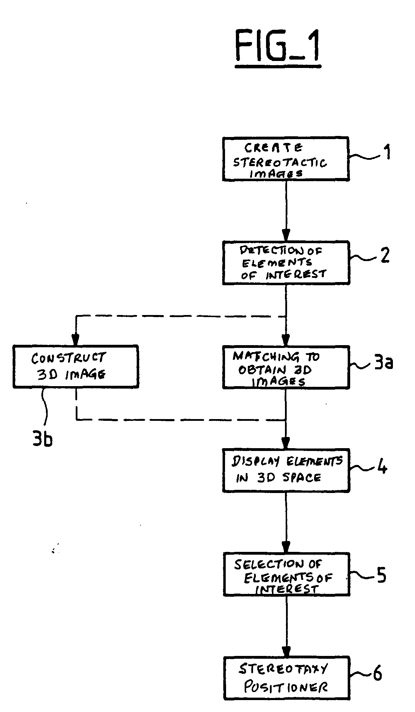

Referring to Figure 1, the different stages that the invention entails can be distinguished. During radiography stage 1, several stereotactic images are made. Stage 2 concerns the automatic detection of elements of interest which are then matched in the course of stage 3a so as to obtain their three-dimensional coordinates. A stage 3b can be carried out at the same time or in place of stage 3a in order to construct a three-dimensional image of a particular element of interest. Stage 4 makes possible the display on the screen of elements of interest in a three-dimensional space, one or more of them are selected in the course of stage 5 and their coordinates are transmitted to a stereotaxy positioner in the course of stage 6.

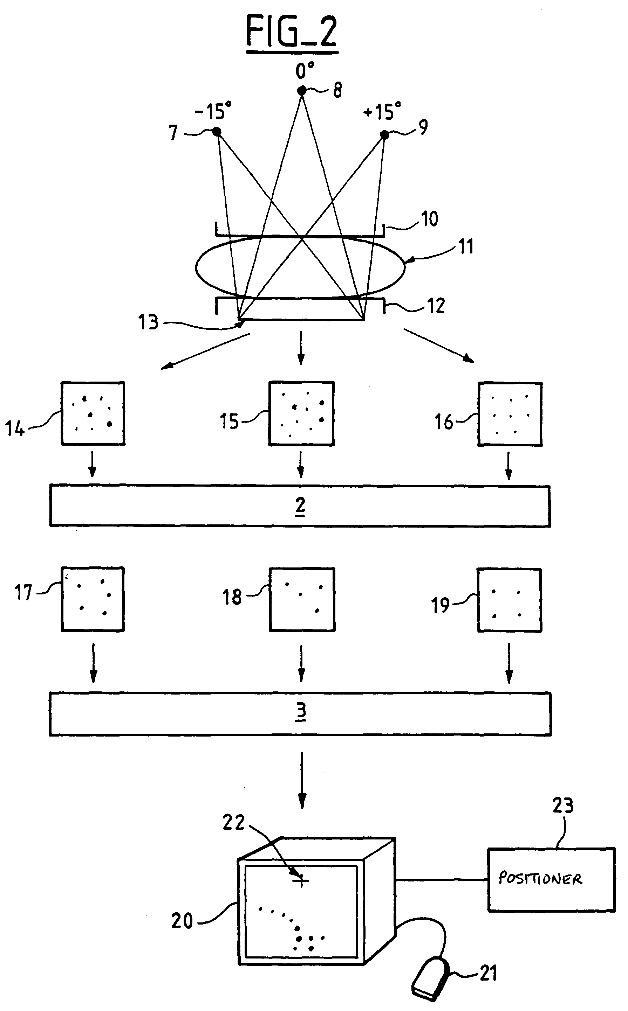

A stereotactic examination, particularly in mammography, consists of a series of two exposures of a three-dimensional object 11 (Figure 2), for example, a breast, resting on a support 12 and compressed by a compression plate 10, by means of an X-ray tube respectively occupying two different positions 7 and 9. In order to center the region of interest of the breast, so that it will be accessible by the biopsy needle through the puncture window, a third exposure is made at the beginning of the procedure by means of the X-ray tube occupying a position 8. A filter (not represented) can be placed at the outlet of the tube so as to select a range of X-rays.

An exposure is made at a 0° angle in order to center the region of interest, and two exposures are made at two angles generally equal and of opposite signs with values typically equal to ±15°. But the invention can also be applied on a number of images greater than three.

A detector 13 makes it possible to capture the X-rays emitted by the sources 7, 8 and 9 and to convert them into visible photons. Three digitized stereotactic images 14, 15 and 16 are thus obtained. These images contain light spots which can be micro-calcifications or parasites, that is, noise occurring in the course of acquisition. According to a convention ordinarily used, the image 14 is the fight-hand image, while image 16 is the left-hand image, and image 15 is the centering image.

At least two of the images thus obtained are going to undergo stage 2 of the automatic detection of micro-calcifications. Several methods of detection of micro-calcifications on a noisy image exist and are known to the expert. To detect the micro-calcifications, a method comprising two phases is used.

There is a first calibration phase in which one determines, from a mathematical model of the acquisition chain and of the breast a plurality of images corresponding to a plurality of entrance gray levels representing a set of parameters of the breast and to a set of parameters of the acquisition chain variable over a range of predetermined values. Then, a mathematical model of detection is elaborated from a system of detection and from the plurality of images, giving a theoretical number of elements detected as a function of a set of parameters of the acquisition chain, of a useful output signal of the system of detection and of a background gray level of the image considered, representing one of the sets of parameters of the breast.

The mathematical model of the acquisition chain and of the breast notably includes a noise model. The acquisition chain contains a detector 13 for which an analysis makes it possible to reveal two main noise sources, the electronic noise of variance $\sigma_e^2$, due to the detector 13, and the quantum noise of the X-rays of variance $\sigma_q^2$.

$\sigma_e$ is a value measured on the detector 13. It is equal to the variance of several images supplied by the detector 13 when the X-ray tube is not emitting any radiation.

The distribution of the noise signal induced by a number N(x,y) of X photons arriving on the detector generally follows a Poisson distribution, but it is evaluated here at a Gaussian distribution of variance equal to N(x,y).

Therefore, $\sigma_q^2 = N(x,y)$ ; x,y are the coordinates of the site on which the X photons arrive on the detector 13.

One then obtains:

$$\sigma_q = \sqrt{N(x,y)}$$

The noise is thus modeled by its variance $\sigma^2$:

$$\sigma^2 = \sigma_q^2 + \sigma_e^2$$

The parameters of the acquisition chain are:

- acquisition parameters: supply voltage of the tube, intensity of the supply current of the tube, exposure time, anode material, exit angle of the X-ray beam, filter material and thickness,

- parameter of a positioner: distance between the tube and the detector 13, rotation angle of the X-ray tube, compression pad material and thickness,

- parameters of the detector 13: type of scintillator, type of photodiode, patient support material and thickness.

This list is not exhaustive and can entail other choices of parameters.

Each gray level presented on input of the mathematical model of the acquisition chain and of the breast advantageously represents a set of parameters of the breast such as its thickness and its composition.

The mathematical model of detection thus established represents the performances of the detection system in relation to a set of parameters variable over a range of given values. In other words, the theoretical response of the system of detection is determined according to all the configurations realizable in the course of an X-ray. The first phase is preferably carried out only once for any detection system.

The second phase is a phase of use in which the system of detection is applied on each stereotactic image. Then, for each elementary zone of a given stereotactic image, one introduces as input data in the mathematical model the useful output signal of the detection system, a set of parameters of the acquisition chain taken among the predetermined values and used to obtain the stereotactic images and a background gray level calculated on the stereotactic image considered, so as to determine a theoretical number of elements detected. One then selects among the elementary zones of the stereotactic image considered those whose theoretical number of elements detected satisfies a predetermined criterion.

In other words, all of the parameters necessary to obtain a theoretical number of elements detected for each elementary zone of the stereotactic image considered are reentered in the mathematical model of detection. These parameters can be separated into three groups, namely, the parameters of the acquisition chain, the output signal of the detector 13 and the parameters calculated from the stereotactic image considered, such as the background gray level.

The system of detection is applied several times on the same image, adapted to each of the sets of parameters of the elements of interest, so as to scan a given range of values of the parameters of the elements of interest.

Their size and shape are taken as parameters of those elements of interest.

The system of detection is then applied several times on the stereotactic image considered, adapted to each of the sets of parameters of the elements of interest, so as to obtain several theoretical numbers of elements detected for each elementary zone of the stereotactic image, and one selects among the elementary zones those whose smallest theoretical number of detected elements obtained satisfies the predetermined criterion.

A top-hat type filter is used as system of detection. The top-hat transformation is a mathematical treatment involving two stages:

1. opening of the image with a structuring element

2. subtraction of the image opened on the original image.

A structuring element is a mask which, applied on an image, makes it possible to delimit a zone of pixels. As far as the top-hat transformation is concerned, when a structuring element is applied on an image, that is, when the mask is placed on each of the elementary zones of the image, all of the structures smaller than the structuring element are eliminated. A given opening corresponds to a structuring element of a given size. In other words, the result of the first stage is a smoothing of the gray levels. The background gray level is thus obtained. The second stage is an operation of subtraction of the background gray levels on the initial image. A high-frequency signal presenting peaks is thus obtained. This type of filtering is known to the expert and the latter may refer, for more details, to the article by J. Serra, *Image Analysis and Mathematical Morphology*, Vol. 2, Academic Press, 1988. In general, this type of filtering makes it possible to extract light or dark small-sized zones from a digital image and to clear the background.

On the high-frequency signal thus obtained, a threshold can then be applied, so as to post a theoretical number of elements of interest detected. Several thresholds are advantageously applied in order to cover a given range of threshold values.

The theoretical number of elements detected is expressed in terms of false positive probability, that is, the probability for an element to be detected when no element of interest has been introduced in the mathematical model of the acquisition chain and of the object.

Satisfying the predetermined criterion entails the establishment of an overall predetermined probability threshold and the selection of elementary zones, in which the sum of the probability values remains less than or equal to the threshold. This criterion can, for example, be maintenance of all the pixels whose false positive probability does not exceed 0.05. These potential elements of interest are represented at 17, 18 and 19. They correspond mainly to microcalcifications.

Stage 3a makes it possible to match the microcalcifications and to determine their three-dimensional coordinates. Several methods of localization of elements of interest exist and are known to the expert. One method of matching and of automatic localization that may be applied to all the microcalcifications is described in the aforementioned French Patent No. 2,751,109. The first stage consists of automatically picking up each microcalcification on each stereotactic image. The microcalcifications being of different size, their central point is taken into account. For each microcalcification, a target pixel is, in fact, selected in a target region of interest present, for example in the right-hand image 14, the target image.

A target window containing the target region of interest is then generated. The target window can be generated automatically after having applied on the target pixel standard software means of growth of region. A rectangular or square target window will then be obtained, the sides of which have dimensions in the order of about ten to several tens of pixels.

The gray levels of each of the pixels of the target window are then stored in a memory associated with a microprocessor which incorporates through software all the functional means of use of the process described.

After that, a set of pixels is selected in a second stereotactic image, for example, the left-hand image 16. This set of selected pixels constitutes a search zone inside which one or more candidate regions of interest likely to be homologous with the target region of interest to be selected.

Then, around each of those pixels selected in the search zone, a second window of the same dimensional characteristic as the target window is generated. The gray levels of the pixels of each second window are stored and a correlation treatment is undertaken between the gray levels of the pixels of each second window and the gray levels of the pixels of the target window. A normed correlation is used for that purpose, in which each correlation value of a second window will then be given by the formula:

$$\frac{\sum_{k=1}^{N} i(k)J(k)}{\sqrt{\sum I^2(k)\sum J^2(k)}} \quad = \quad \frac{\vec{I} \cdot \vec{J}}{\left\|\vec{I}\right\| \left\|\vec{J}\right\|}$$

where N is the number of pixels of a window, k the current index of a pixel, I(k) the gray level of the pixel k of the target window, and J(k) the gray level of the pixel k of the second window.

The correlation treatment therefore makes it possible to take a measurement of similarity between the target window and each of the second windows of the second image so as to obtain a similarity value, that is, a value of correlation between those two windows.

Matching of the target region of interest with its homologous region of interest is made by analysis of these correlation values.

A restricted set of peak correlations is then selected among the set of correlation values according to a predetermined criterion like peak intensity or number of peaks in the list of correlation values arranged in order of decreasing intensity. The peak correlation can be used, for example, as matching criterion between the target window and one of the second windows of the second image. The three-dimensional coordinates of the target microcalcification is deduced therefrom by a trigonometric calculation, knowing the geometry of the stereotactic device.

A predefined number $N_1$ of candidate regions of interest likely to be the region homologous with the target region appearing on the right-hand image 14 can also be selected, for example, on the left-hand image 16. This selection is therefore made automatically on the $N_1$ first peak correlations.

With the target pixel of the target region of interest and the candidate pixel having given rise to selection of the candidate region of interest, the three-dimensional, coordinates of the corresponding point of the candidate microcalcification corresponding to those $N_1$ regions are determined by a stereotactic trigonometry calculation, knowing the geometry of the stereotactic device. One then determines, by an inverse stereotactic trigonometry calculation, the coordinates in the centering image of the projected pixel corresponding to projection of the pixel of the candidate element in that centering image.

One therefore takes among the $N_1$ candidate regions the one whose projection on the centering image is superposed on a region of interest physically present in the centering image.

This process of matching is carried out on the all of the microcalcifications determined on the stereotactic images. One therefore possesses the three-dimensional coordinates of each microcalcification. The same microcalcification can be visible on more than two stereotactic images. The microcalcifications can advantageously be classified according to their correlation value obtained in relation to all of the stereotactic images. That is, the microcalcification which is visible on all the images will have a total correlation value higher than the one visible only on two images.

One can then take, for example, a predefined number $N_2$ of microcalcifications corresponding to the $N_2$ greatest total correlation values. Their coordinates are sent to display software. The software makes it possible to construct a three-dimensional image consisting of $N_2$ microcalcifications positioned identically to their position in the breast. The image can be visible, in the course of stage 4, on a microcomputer screen 20, for example.

This software incorporates functions of rotation, translation and cursor 22, so that it is possible to observe the microcalcifications at any angle. It then becomes possible to observe their possible alignments not always visible on two-dimensional stereotactic images, notably because these microcalcifications are superposed. These alignments can correspond to milk ducts, and this then denotes the origin of the microcalcifications and therefore provides additional diagnostic information in relation to the projection images. This software is included in a microcomputer which is directly connected to a positioner 23.

The selection, that is, the choice of user, takes place in the course of stage 5. By clicking on a microcalcification pointed to by the cursor 22 by means of a mouse 21, the coordinates of the microcalcification are transmitted in the course of stage 6 (Figure 1) to the positioner 23, in order to guide the puncture needle in the breast to the site actually pointed to on the screen.

A variant of the invention resides in the fact that, following the determination of microcalcifications in the course of stage 2, in place of stage 3a in which the three-dimensional coordinates are determined, one can also take a microcalcification (or a cluster of microcalcifications) which seems to be of particular interest for display in the course of stage 4 in its real form. For this purpose, a stage 3b is carried out for reconstruction of a three-dimensional image from several two-dimensional images. In this case, it is advantageous to make the reconstruction from a large number of images, for example, six. The reconstruction makes it possible to obtain a three-dimensional envelope of the element of interest. It brings into play the acquisition geometry and calculation of the three-dimensional intersection of a set of cones generated by X-radiation of the contours of the element of interest. This technique of three-dimensional reconstruction of an object from contours is known to the expert. However, the expert may possibly refer to the document entitled "Localization and three-dimensional reconstruction from stereotactic angiograms," Doctoral Thesis, National Polytechnic Institute of Lorraine, L. Launay, December 1996.

The shape of the element of interest can then be displayed by means of the same display software. The user can thus observe it at different angles and view possible zones which do not contain elements of interest and would not be visible on two-dimensional images.

This contribution to the decision-making process introduces the user's choice only at the last moment, that is, once the three-dimensional representation is established. The three-dimensional representation makes it possible to enrich the diagnosis, for it supplies information practically inaccessible with two-dimensional stereotactic images. Thus, the user can calmly make his/her choice as to the region to be punctured.

**Claims**

1. A method of localization and representation of elements of interest of an organ from a plurality of digital images taken at different angles, comprising the steps of:

    (a) the elements of interest present in the digital images are determined;

(b) the thee-dimensional coordinates of each element of interest are then determined;

(c) the elements of interest are displayed in three dimensions; and

(d) one or more elements of interest are selected manually.

2. The method according to claim 1, wherein the elements of interest are determined by means of an automatic process of detection of elements of interest.

3. The method according to claim 2, wherein the automatic process of detection employs a mathematical model of the acquisition chain and of the organ to be X-rayed.

4. The method according to claim 2, wherein the automatic process of detection employs a noise model.

5. The method according to claim 2, wherein the automatic process of detection takes into account the acquisition parameters of the acquisition chain.

6. The method according to claim 2, wherein the automatic process of detection uses a top-hat transformation.

7. The method according to any one of the foregoing claims, wherein the thee-dimensional coordinates of each element of interest detected are determined by making an automatic matching from at least two of the digital images.

8. The method according to any one of the foregoing claims, wherein the three-dimensional coordinates of one or more selected elements of interest are sent to a needle positioner.

9. The method according to claim 1, wherein following determination of the elements of interest, a reconstruction of three-dimensional images of an element of interest is made from two-dimensional images in which that element of interest has been detected, and its three-dimensional form is then displayed.

10. The method according to any one of the foregoing claims, wherein a system of on-screen display integrating a rotation function is used, in order to distinguish the alignment of the elements of interest upon a diagnosis.

# FIG_1

```
┌─────────────────┐
│    CREATE       │ ~1
│  STEREOTACTIC   │
│    IMAGES       │
└─────────────────┘
         │
         ▼
┌─────────────────┐
│  DETECTION OF   │ ~2
│   ELEMENTS      │
│  OF INTEREST    │
└─────────────────┘
         │
    ┌ ─ ─│─ ─ ─ ─ ─ ─ ─ ─ ─ ─ ┐
    │    ▼
┌─────────┐        ┌─────────────────┐
│CONSTRUCT│        │  MATCHING TO    │ ~3a
│3D IMAGE │        │  OBTAIN 3D      │
└─────────┘        │   IMAGES        │
    │              └─────────────────┘
   3b                       │
    └ ─ ─ ─ ─ ─ ─ ─ ─ ─ ─   ▼
          ┌─────────────────┐
          │ DISPLAY ELEMENTS│ ~4
          │  IN 3D SPACE    │
          └─────────────────┘
                   │
                   ▼
          ┌─────────────────┐
          │  SELECTION OF   │ ~5
          │  ELEMENTS OF    │
          │   INTEREST      │
          └─────────────────┘
                   │
                   ▼
          ┌─────────────────┐
          │  STEREOTAXY     │ ~6
          │  POSITIONER     │
          └─────────────────┘
```

# FIG_2

**European Patent Office**

## EUROPEAN SEARCH REPORT

Application Number

EP 00 30 3309

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 803 912 A (BERNARD SICZEK ET AL.) 8 September 1998 (1998-09-08) * column 4, line 5 - line 61; claim 1 * * column 5, line 26 - line 36 * * column 8, line 58 - column 9, line 8 * ----- | 1,7,8 | G06T7/00 A61B6/00 |

|  |  |
|---|---|
|  | **TECHNICAL FIELDS SEARCHED (Int.Cl.7)** |
|  | G06T A61B |

The present search report has been drawn up for all claims

| Place of search | Date of completion of the search | Examiner |
|---|---|---|
| THE HAGUE | 30 June 2000 | Chateau, J-P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

## ANNEX TO THE EUROPEAN SEARCH REPORT
## ON EUROPEAN PATENT APPLICATION NO.

EP 00 30 3309

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2000

| Patent document cited in search report | Publication date | Patent family member(s) | Publication date |
|---|---|---|---|
| US 5803912          A | 08-09-1998 | US    5415169 A<br>US    5078142 A<br>US    5240011 A<br>US    6022325 A<br>US    5735264 A<br>EP    0625024 A<br>WO    9310710 A | 16-05-1995<br>07-01-1992<br>31-08-1993<br>08-02-2000<br>07-04-1998<br>23-11-1994<br>10-06-1993 |

EPO FORM P0459

For more details about this annex : see Official Journal of the European Patent Office, No. 12/82